# EUROPÄISCHE PATENTSCHRIFT

(11) **EP 1 164 978 B1**
(45) Veröffentlichungstag und Bekanntmachung des Hinweises auf die Patenterteilung: **31.12.2008**
(21) Anmeldenummer: 00925184.4
(22) Anmeldetag: 07.04.2000
(51) Int. Cl.: A61F 2/36, A61F 2/30

(54) **BLATTARTIGER SCHAFT EINER HÜFTGELENKPROTHESE FÜR DIE VERANKERUNG IM FEMUR**
FLAT SHAFT OF A HIP JOINT PROSTHESIS FOR ANCHORING IN THE FEMUR
TIGE PLATE D'UNE PROTHESE DE ROTULE DESTINEE A ETRE INSEREE DANS LE FEMUR

(30) Priorität: 07.04.1999 DE 19915677; 23.06.1999 DE 19928790
(43) Veröffentlichungstag der Anmeldung: 02.01.2002
(73) Patentinhaber: Smith & Nephew Orthopaedics AG, 6343 Rotkreuz (CH)
(72) Erfinder: ZWEYMÜLLER, Karl, A-1190 Wien (AT)
(74) Vertreter: Popp, Eugen
(86) Internationale Anmeldenummer: PCT/EP2000/003143
(87) Internationale Veröffentlichungsnummer: WO 2000/059410

(56) Entgegenhaltungen:
- EP-A- 0 700 670
- EP-A- 0 720 839
- FR-A- 2 634 642
- FR-A- 2 639 821
- US-A- 5 593 451

## Beschreibung

Die Erfindung betrifft eine Kombination von einem blattartigen Schaft einer Hüftgelenkprothese für die Verankerung im Femur und einer dazu-gehörigen Raspel nach dem Oberbegriff des Anspruches 1.

Eine derartige Kombination ist aus der EP 0 032 165 B1 bekannt. Bei dieser bekannten Konstruktion erweitert sich der Schaft vom distalen Ende aus in Richtung seiner Längsachse bis in einen Bereich zwischen etwa 2/3 und 3/4 der Schaftlänge - gemessen entlang der Längsachse - etwa allseitig konisch. Die mediale Schmalseite des Schaftes geht aus dem Konus heraus in einen stetig gekrümmten Bogen über, der an einem kragenartigen Ansatz endet. Dieser Ansatz trennt einen Femur-Verankerungsabschnitt des Schaftes von einem Prothesenhals, der einen sich nach proximal konisch verjüngenden Zapfen umfaßt, welcher zur Aufnahme eines kugelförmigen Gelenkkopfes dient. Die Prothesenhalsachse schneidet die Längsachse des Schaftes unter einem Winkel, der im wesentlichen dem Winkel zwischen dem Schenkelhals und der Femurachse eines natürlichen Hüftgelenkes entspricht.

Die bekannte Konstruktion hat sich bewährt, um eine sogenannte "Schlußrotation" beim Einsetzen der Prothese zu vermeiden, ohne die Resektionsebene am Schenkelhals zu tief ansetzen zu müssen. Diese nachteilige "Schlußrotation" besteht darin, daß es beim vollständigen Einsetzen der konventionellen Prothesenschäfte wegen der erforderlichen Mindestdicke des Schaftblattes häufig zu einer Rotation des Schaftes im Femurknochen kommt, da infolge der mehrfachen Krümmungen des proximalen Femurendes ein gerader oder auch leicht gebogener Gegenstand von der Wand des Oberschenkelknochens abgelenkt wird.

Der vorliegenden Erfindung liegt die Aufgabe zugrunde, eine verbesserte gattungsgemäße kombination anzugeben, beider der Schaft im proximalen Bereich besser verankert werden kann.

Diese Aufgabe wird durch eine kombination mit den Merkmalen des Anspruchs 1 gelöst.

Die Erfindung schließt den Gedanken ein, den Schaft in seinem proximalen Bereich in der anterior-posterioren Erstreckung und gegebenenfalls auch in der medial-lateralen Erstreckung, bezogen auf das sogenannte "Raspelmaß" oder "Nullmaß" (mit dem die Ausnehmung im Femur zur Aufnahme des Schaftes ausgearbeitet wird), mit einem geringfügigen Übermaß auszubilden, während er im mittleren und distalen Abschnitt im wesentlichen paßgenau zur geraspelten Markraumhöhle gefertigt ist. Durch diese Maßnahme wird eine dosierte flächige bzw. volumenmäßige Spongiosaverdichtung und gewissermaßen ein Preßsitz des Schaftes im proximalen Bereich des Femurs erreicht. Gegenüber bekannten Anordnungen mit proximalen Rippen, die sich in den Femur schneiden und hohe Spannungsspitzen erzeugen, die die Gefahr eines Sprengens des Knochens mit sich bringen, ist diese Art der Verdichtung wesentlich unkritischer. Das Entstehen eines Spaltes zwischen dem Schaft und dem geraspelten Hohlraum wird sich vermieden, wobei zugleich im distalen Bereich ein exaktes Raspeln an/in die Kortikalis ermöglicht wird.

Eine vorteilhafte Realisierung dieses Gedankens besteht darin, im proximalen Endbereich des Schaftes an mindestens einer seiner Flachseiten neben der üblichen "konischen" Erweiterung eine zusätzliche Erweiterung bzw. Verdickung vorzusehen.

Durch diese konstruktive Maßnahme wird ein verbesserter Sitz des Schaftes im proximalen Bereich des Femur erhalten, ohne daß die Gefahr eines Femurbruchs besteht. Zu diesem Zweck ist die zusätzliche "konische" Erweiterung nach proximal relativ gering ausgebildet, jedoch ausreichend, um den gewünschten verbesserten Halt des Schaftes im Femur zu erreichen. Um das genannte Ziel ohne allzu große zusätzliche Belastung des Femurs zu erreichen, liegt das erwähnte Übermaß bei 1-3 %. Vorteilhaft ist, wenn die maximale Höhe der zusätzlichen Erweiterung des Schaftes im proximalen Bereich 2/10 mm bis etwa 10/10 mm beträgt.

Die vorgeschlagene Übermaßigkeit, speziell in Form der erwähnten zusätzlichen Erweiterung ("Konus"), wird vorzugsweise durch eine zusätzliche Beschichtung des Schaftes im proximalen Bereich, insbesondere mittels eines Plasmabeschichtungsverfahrens mit Titan oder mit Hydroxylapatit, erhalten. Für diese Beschichtung hat sich ein Aufbau aus mehreren Schichten als vorteilhaft erwiesen, insbesondere ein Aufbau mit einer dichten, relativ dünnen Grundschicht und einer erheblich dickeren, porösen Deckschicht oder Zwischenschicht und wahlweise einer auf der porösen Zwischenschicht angeordneten dichten Hydroxylapatitschicht.

Der Punkt auf der Länge des Schaftes, an dem die zusätzliche Erweiterung beginnt, kann anterior und posterior gleich, aber auch unterschiedlich gewählt sein, und die Verdickung kann ab diesem Punkt nach proximal eine stetig anwachsene Höhe aufweisen oder auch als stufige Verdickung ausgeführt sein.

Um einerseits eine zu starke Belastung des Femurknochens zu vermeiden, andererseits jedoch den gewünschten verbesserten Halt des Schaftes zu erreichen, ist es besonders vorteilhaft, wenn der Abschnitt der zusätzlichen Erweiterung in Längsrichtung (in Richtung nach proximal) lateral konkav gewölbt sowie im Schnitt senkrecht zur Schaftlängsachse etwa spitzdachförmig ausgebildet ist.

Ein weiterer wesentlicher Aspekt besteht in einer im Querschnitt abgeflachten Ausbildung auch des Prothesenhalses. Dessen Querschnittsgestalt wird dabei bis zu einem gewissen Grade an den blattartigen Querschnitts des Femur-Verankerungsabschnitts angeglichen; das Spektrum umfaßt mehr oder minder flache Ellipsen, in den Eckbereichen abgerundete Rechtecke, Kombinationen aus Kreisabschnitten und Geraden oder ggf. auch den nachfolgend näher beschriebenen Schaftquerschnitten ähnliche Formen mit "spitzdachartigen" Seitenflächen.

Noch ein weiterer wesentlicher, in Kombination mit der vorgeschlagenen zusätzlichen anterioren und/oder posterioren Erweiterung im proximalen Bereich besonders vorteilhafter Aspekt der Erfindung besteht im Vorsehen einer differenzierten Oberflächenausführung über die Schaftlänge: Das distale Schaftende (ein Bereich von etwa 1/5 der Schaftlänge) wird poliert ausgeführt, während der übrige Bereich die übliche Rauhigkeit - die in herkömmlicher Weise durch Korundstrahlen erzielt wird - aufweist oder nochmals in zwei Zonen unterteilt ist. Im letzteren Falle wird bevorzugt im proximalen Bereich eine grobe, offenporige Struktur mit besonders hoher Rauhigkeit vorgesehen, während der mittlere Abschnitt des Prothesenschaftes keine offenen Poren und die erwähnte mittlere oder übliche Rauhigkeit aufweist.

Besonders hervorzuheben ist noch die Ausbildung einer sogenannten Facette im proximalen Bereich, d. h. im Übergangsbereich zwischen medial einerseits und posterior und anterior andererseits bzw. zwischen lateral einerseits und posterior und anterior andererseits. Das Vorsehen dieser Facetten gewinnt im Zusammenhang mit der vorgeschlagenen Übermaßigkeit des Schaftes im proximalen Bereich eine spezielle Bedeutung. In den Kantenbereichen des Schaftes würde die Übermaßigkeit nämlich die Gefahr einer übermäßigen Beanspruchung der Corticalis mit sich bringen, so daß dort eine hohe Paßgenauigkeit gesichert werden soll. Daher werden die Kantenbereiche durch nachträgliches Fräsen auf das genaue Raspelmaß gebracht. Besonders vorteilhaft ist eine abgestufte, abgerundete oder zusätzlich abgeschrägte Ausführung der Anfasungsbereiche zu den Seitenflächen hin, die weiter unten genauer erläutert wird.

In einer speziellen, vorteilhaften Ausführung hat der Schaftquerschnitt mindestens im proximalen Bereich annähernd eine Trapezform, insbesondere eine symmetrische Trapezform mit zwei gleich langen längeren Seitenkanten a, die im Querschnitt die anterior bzw. posterior gelegenen Seitenflächen des Schaftes begrenzen, und zwei unterschiedlich langen kürzeren Seitenkanten, von denen die kürzere der medialen Stirnfläche und die längere der lateralen Stirnfläche des Schaftes entspricht.

Weitere vorteilhafte konstruktive Maßnahmen und Alternativen der erfindungsgemäßen Konstruktion sind in den Unteransprüchen sowie den folgenden Ausführungsbeispielen beschrieben.

Nachstehend werden Ausführungsbeispiele anhand der beigefügten Zeichnungen näher erläutert. Diese zeigen in
- Fig. 1: eine Stirnseitenansicht eines Schaftes gemäß einer Ausführungsform der Erfindung von lateral;
- Fig. 2: eine Ansicht des Schaftes nach Fig. 1 von anterior oder posterior;
- Fig. 3 - 6: Ausführungsformen eines erfindungsgemäß ausgebildeten Schaftes mit verschiedenen Querschnitten längs der Linie B-B in Fig. 2;
- Fig. 7 und 8: weitere alternative Querschnittsformen des erfindungsgemäßen Schaftes;
- Fig. 9: eine Teil-Seitenansicht einer weiteren Ausführungsform (proximaler Bereich) von anterior oder posterior,
- Fig. 10: eine Seitenansicht eines Schaftes gemäß einer weiteren Ausführungsform sowie
- Fig. 11 und 12: Querschnittsdarstellungen dieses Schaftes längs der Schnittlinien A-A bzw. B-B in Fig. 10 und
- Fig. 13 - 16: verschiedene Prothesenhals-Querschnittsformen.

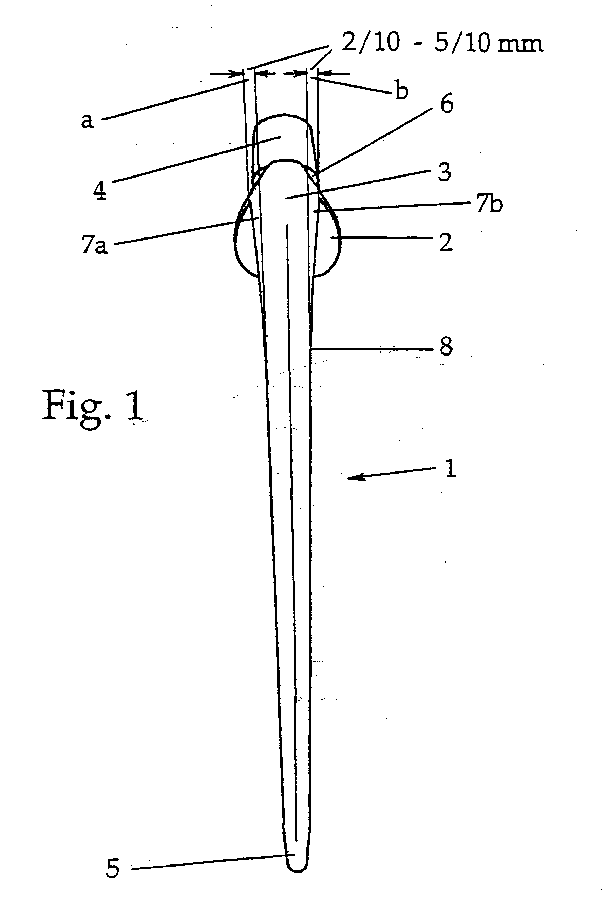

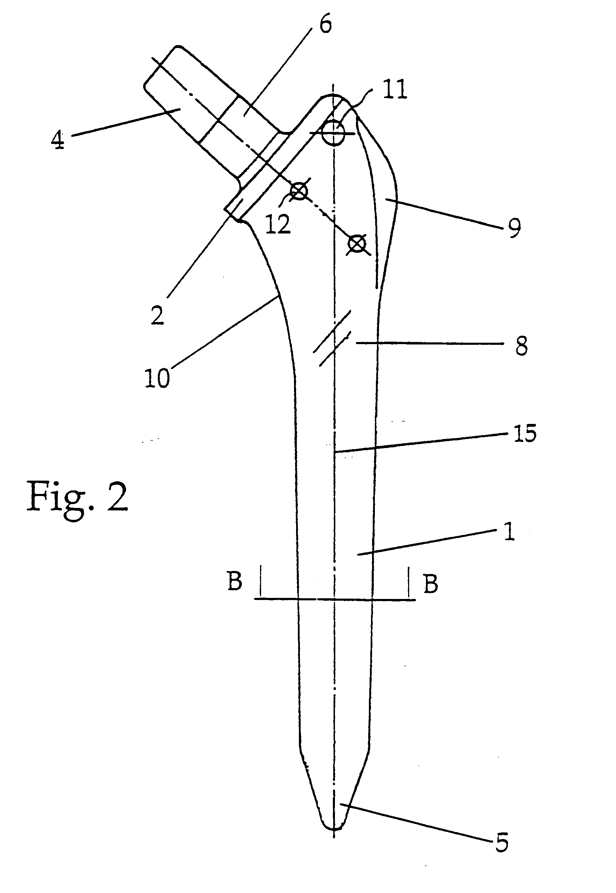

Ein in den Fig. 1 und 2 dargestellter Schaft 1 dient zur Verankerung einer Hüftgelenkprothese im Femur. Wie die Fig. 1 und 2 erkennen lassen, erweitert sich der Schaft, ausgehend von einem distalen Ende 5, in Richtung nach proximal bis hin zu einem kragenartigen Ansatz 2, der einen Verankerungsabschnitt des Schaftes 1 von einem Prothesenhals 6 trennt, allseitig etwa gleichmäßig. Entsprechend Fig. 2 verläuft die mediale Schmalseite des Schaftes aus dem "Konus" heraus in einem stetig gekrümmten Bogen 10, der an dem erwähnten Ansatz 2 endet. Mit der Bezugsziffer 3 ist ein oberer Übergangsbereich gekennzeichnet, mit Ziffer 9 (Fig. 2) ein Trochanterflügel, und mit den Ziffern 11 und 12 Querbohrungen, die den Schaft anterior nach posterior durchsetzen. Diese Bohrungen haben die Funktion, den Vergleich von zu verschiedenen Zeiten hergestellten Röntgenaufnahmen zu erleichtern. Es läßt sich auf diese Weise auch ein möglicher Versatz des Schaftes nach einem länger zurückliegenden Einsatz erkennen. Die Schaftlängsachse (Längsmittelachse) ist in Fig. 2 mit der Bezugsziffer 15 gekennzeichnet. Der oben erwähnte, sich konisch verjüngende Zapfen, der sich an den Prothesenhals 6 anschließt, ist mit Bezugsziffer 4 gekennzeichnet.

Wie Fig. 1 erkennen läßt, verläuft die Erweiterung des Schaftes 1 an der anterioren und posterioren Seite zunächst stetig bis zu einem proximalen Bereich 8. Im proximalen Bereich 8 ist die oben erwähnte durchgehende allseitige Erweiterung ("Konus") durch einen zusätzlichen "Konus" 7a, 7b sowohl an der anterioren als auch posterioren Seite überlagert, in dessen Bereich die Seitenflächen beispielsweise gewölbt sind. Die maximalen Höhen der zusätzlichen Erweiterung "a", "b" erreichen Werte von 2/10 mm bis 5/10 mm. Sie können anterior und posterior gleichermaßen ausgebildet sein. Es ist jedoch auch denkbar, die Höhen "a" und "b" posterior und anterior unterschiedlich zu wählen.

Es hat sich, wie eingangs erwähnt, herausgestellt, daß durch die zusätzliche Erweiterung 7a, 7b eine erhebliche Verbesserung der Verankerung des Schaftes 1 im proximalen Bereich erreicht wird, ohne daß der Femur dadurch übermäßig beansprucht wird.

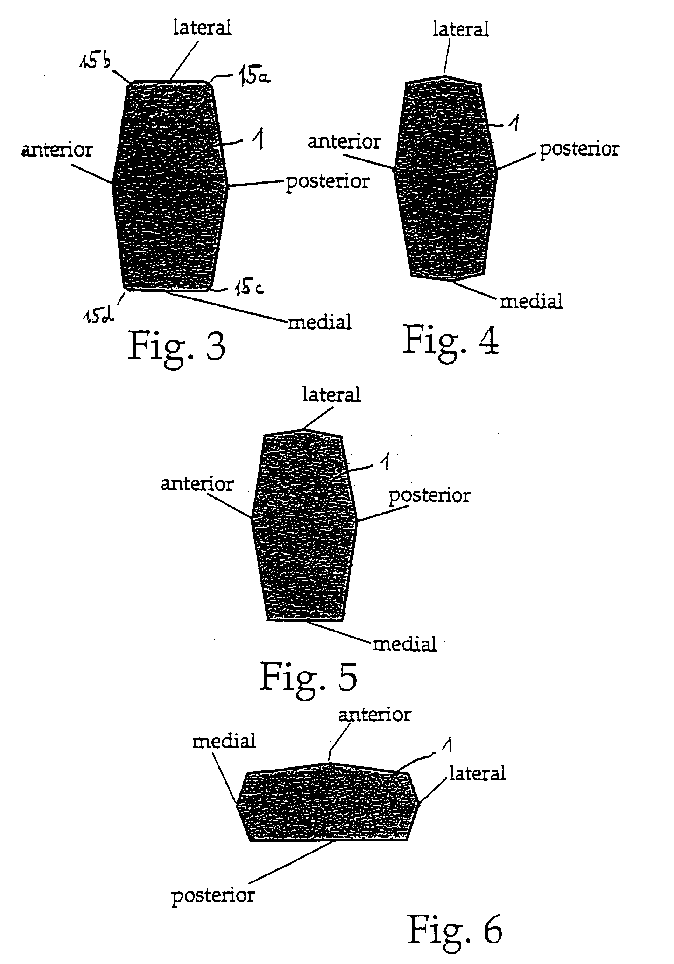

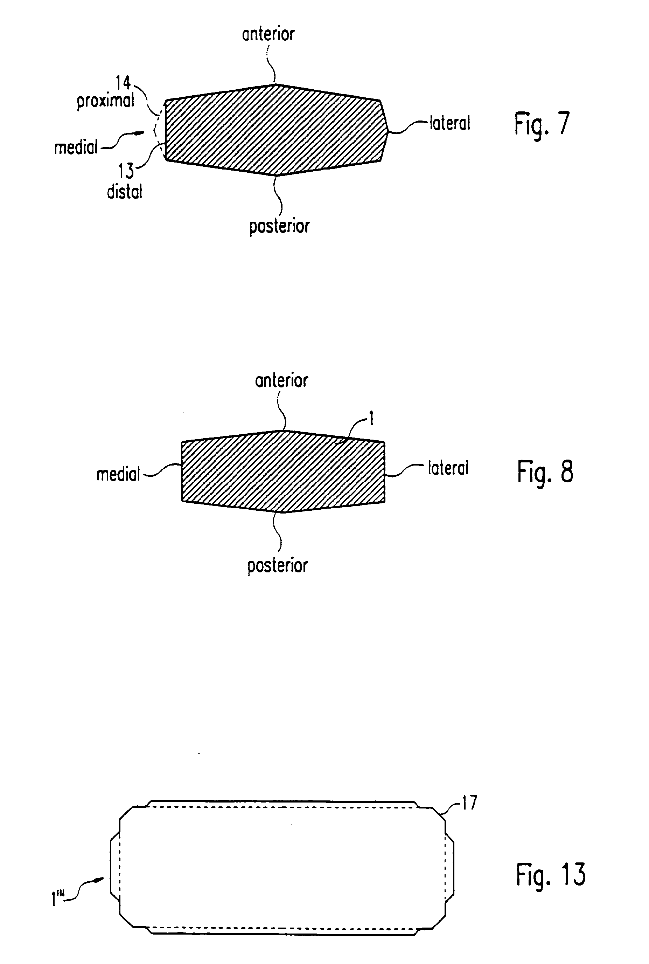

Wie die Fig. 3 - 8 sehr gut erkennen lassen, kann der Schaft 1 bzw. dessen Femur-Verankerungsabschnitt unterschiedliche Querschnittsformen aufweisen. Die Fig. 3 - 8 zeigen Querschnittsformen des Schaftes 1 etwa auf Höhe der Linie B-B in Fig. 2. Die dargestellten Querschnittsformen liegen entweder durchgehend über die gesamte Schaftlänge oder auch nur im distalen oder proximalen Bereich vor. In speziellen Ausführungen hat im distalen Bereich der Schaft einen Querschnitt entsprechend Fig. 5, im proximalen Bereich einen Querschnitt entsprechend Fig. 4. Insofern lassen sich die Querschnitte gemäß den Fig. 3 - 8 unterschiedlich kombinieren je nach den individuellen Verhältnissen. Fig. 7 deutet eine solche Kombination zweier unterschiedlicher Querschnitte an. An der medialen Seite des Schaftes 1 ist dieser im distalen Bereich flach ausgebildet (mediale Flachseite 13), während im proximalen Bereich die mediale Seite in eine Spitzdach-Konfiguration 14 übergeht.

Wie schon erwähnt, können natürlich auch andere Querschnitts-Kombinationen gewählt werden. Beispielsweise kann gemäß Fig. 6 die posteriore Seite flach, die anteriore Seite spitzdachförmig gestaltet sein. Der in Fig. 7 angedeutete Übergang an der medialen Seite von distal nach proximal kann in ähnlicher Weise an der posterioren und/oder anterioren Seite ausgebildet sein.

Konkret zeichnen sich die unterschiedlichen dargestellten Querschnitte wie folgt aus:
- Fig. 3:: lateral und medial flach anterior und posterior flach spitzdachförmig
- Fig. 4:: lateral, medial, anterior und posterior jeweils spitzdachförmig
- Fig. 5:: lateral, anterior und posterior spitzdachförmig medial flach
- Fig. 6:: medial, lateral und anterior spitzdachförmig posterior flach
- Fig. 7:: anterior, posterior und lateral spitzdachförmig medial distal flach und proximal spitzdachförmig
- Fig. 8:: anterior und posterior spitzdachförmig medial und lateral flach.

Bei der in Fig. 3 gezeigten Ausführungsform sind die Kanten bzw. Übergangsbereiche zwischen lateral und medial einerseits sowie anterior und posterior andererseits jeweils abgeschrägt bzw. angefast. Die entsprechenden Facetten sind mit den Bezugsziffern 15a, 15b, 15c und 15d gekennzeichnet.

Diese Facetten schonen den Femur innenseitig einerseits; andererseits füllen sie auch den Femur innenseitig besser aus.

Der Facettenwinkel beträgt in der Regel etwa 45° zur Längsmittenebene parallel zur anterioren bzw. posterioren Seite des Schaftes 1. Dieser Winkel kann über die Schaftlängsachse durchgehend konstant ausgebildet sein. In einer alternativen Ausführung ist dieser Winkel im proximalen Bereich in Richtung von distal nach proximal zunehmend flacher gestaltet.

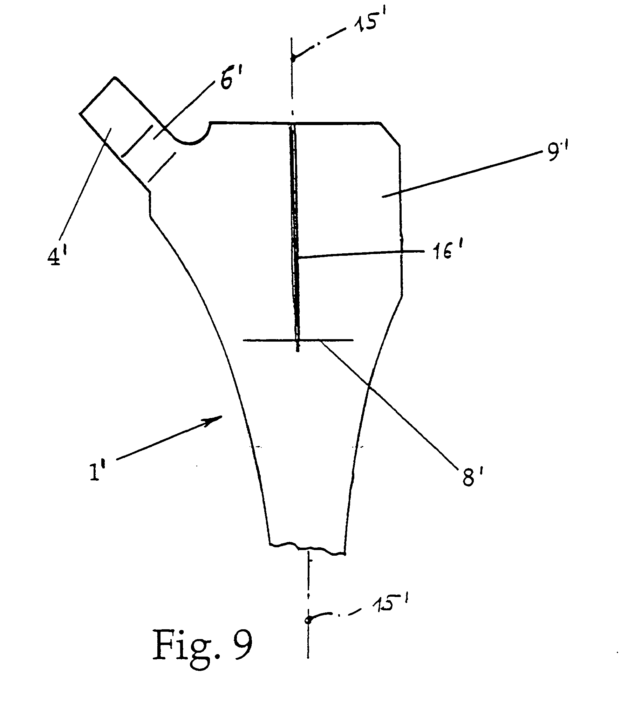

Fig. 9 zeigt eine abgewandelte Ausführungsform eines Schaftes 1', und zwar in Ansicht von posterior oder anterior, wobei in Fig. 9 nur der proximale Bereich dargestellt ist. Mit dem Schaft nach Fig. 1 übereinstimmende Elemente bzw. Abschnitte sind mit an Fig. 1 angelehnten Bezugsziffern bezeichnet und werden nachfolgend nicht nochmals erläutert.

In dieser Ausführungsform ist kein kragenartiger Ansatz zwischen Verankerungsabschnitt und Prothesenhals 6' vorgesehen. Dieser Schaft zeichnet sich durch einen ausgeprägten Trochanterflügel 9' aus, der auch bei dem Schaft gemäß den Fig. 1 und 2 vorgesehen ist, jedoch in einer weniger ausgeprägten Form.

An einem "Dachfirst" im proximalen Bereich, der mit der Bezugsziffer 16 gekennzeichnet ist, ist erkennbar, daß der proximale Bereich des Schaftes, der etwa an der Linie 8' beginnt, posterior und/oder anterior flach spitzdachförmig ausgebildet ist. Distal der Linie 8 nehmen die anteriore und posteriore Seitenfläche des Schaftes plane Gestalt an.

Der Schaftquerschnitt kann an der medialen, lateralen, posterioren und/oder anterioren Seite konkav oder konvex gewölbt sein, zumindest bereichsweise (beispielsweise durch jeweils konkave oder konvexe Krümmung der Flächen des "Spitzdaches").

In einer weiteren speziellen Ausgestaltung sind die erwähnten Seiten des Schaftes zumindest bereichsweise mit Längsrillen und/oder Längsrippen versehen, die im Querschnitt jeweils etwa dreieck-, spitzdach- oder rechteckförmig sein können.

Selbstverständlich ist der beschriebene Schaft aus einem humanverträglichen Material, insbesondere einer Titanlegierung, hergestellt. Die Oberflächen können zusätzlich aufgerauht sein.

In einer bevorzugten Ausführung ist ein sich über etwa ein Fünftel der Gesamtlänge erstreckender distaler Endbereich des Prothesenschaftes poliert, der sich daran anschließende mittlere Bereich in üblicher Weise durch Korundstrahlen mit einer mittleren Rauhigkeit versehen und der proximale Endbereich mit besonders großer Rauhigkeit offenporig ausgebildet. Zusammen mit der zusätzlichen Erweiterung im proximalen Bereich wird durch diese abgestufte Oberflächenrauhigkeit, mit der differenzierte Grenzschichtbedingungen für das Einwachsen in den Femur realisiert werden, eine im proximalen Bereich besonders feste Verankerung erzielt.

In einer speziellen Variante ist der Längsverlauf der zusätzlichen Erweiterung an der posterioren und/oder anterioren Seite des Schaftes statt konkav gekrümmt geradlinig ausgebildet. Bei konkav gekrümmter Ausbildung kann die Wölbung statt eines Kreisbogenabschnitts einen Parabel-, Hyperbel- oder Ellipsenabschnitt umfassen. Grundsätzlich ist auch ein konvex gekrümmter oder S-förmiger Längsverlauf ausführbar.

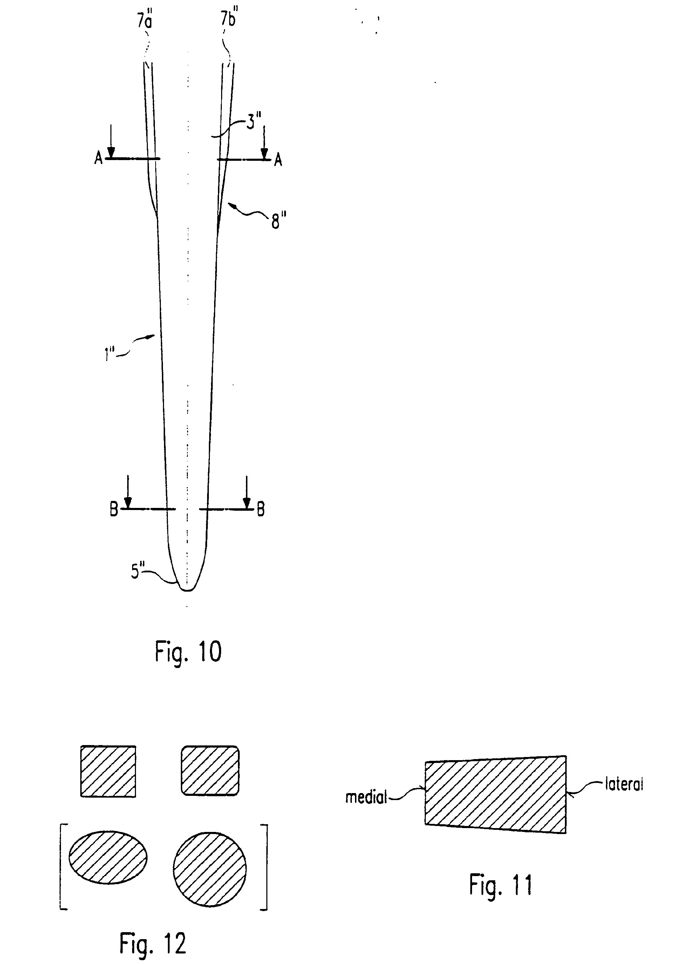

Fig. 10 zeigt eine Teilansicht einer weiteren Ausführungsform eines Prothesenschaftes 1", der grundsätzlich den in Fig. 1 und 2 bzw. 9 dargestellten Schäften ähnlich ist und bei dem daher die Bezugsziffer zur Bezeichnung bestimmter Elemente bzw. Bereiche an die erwähnten Figuren angelehnt sind. Der Schaft 1" zeichnet sich zunächst dadurch aus, daß in seinem proximalen Bereich 8" die anteriore bzw. posteriore Verdickung 7a" bzw. 7b" an Punkten mit unterschiedlichem Abstand zum distalen Ende 5" des Schaftes einsetzt. Weiterhin ist zu erkennen, daß der Verlauf der Verdickung 7b" ab ihrem Einsatzpunkt zunächst flacher als bei der Verdickung 7a" ist. Die Verdickungen bzw. Erweiterungen 7a", 7b" sind durch einen Mehrschichtaufbau aus einer dichten Grundschicht von ca. 50 µm Dicke, einer porösen Zwischenschicht von ca. 300 µm Dicke und einer dichten Hydroxylapatit-Deckschicht von ca. 50 µm Dicke gebildet (diese Dickenangaben sind lediglich Beispielwerte und können zur Erzielung eines gewünschten Übermaßbetrages des Schaftes im proximalen Bereich erheblich variieren).

In den Figuren 11 und 12 sind bevorzugte Schaftquerschnitte längs der Schnittlinie A-A bzw. B-B in Fig. 10 gezeigt. Der Schaftquerschnitt im proximalen Bereich ist - in Fig. 11 vereinfacht ohne Berücksichtigung der zusätzlichen Beschichtung skizziert - im wesentlich symmetrisch trapezförmig, wobei die mediale Stirnseite des Schaftes in Anpassung an die anatomischen Gegebenheiten schmaler als die laterale Stirnseite ist. Im distalen Bereich kann der Schaftquerschnitt (im Unterschied zu den in der unteren Zeile von Fig. 12 dargestellten herkömmlichen Schaftquerschnitten) im wesentlichen quadratisch oder rechteckig sein. Die Kantenbereiche können - ebenso wie bei der trapezförmigen Querschnittsform im proximalen Bereich - abgerundet oder insbesondere angefast sein, was in den Figuren 11 oder 12 jedoch nicht dargestellt ist.

Auf der Grundlage der trapezförmigen Grundform nach Fig. 10 sind vielfältige Modifikationen ausführbar, etwa ein asymmetrisches Schrägkreuz, ein "H" mit einem längeren und einem kürzeren Schenkel, eine "Doppel-H"-artige Ausführung mit drei unterschiedlich langen Schenkeln, eine Ausführung mit Hohlfacetten in den Kantenbereichen eines Trapez-Querschnitts oder verschiedene Hohlprofile mit trapezförmiger Außengestalt.

In Fig. 13 ist zur Darstellung einer weiteren speziellen Ausführung des Verankerungsabschnitts einer erfindungsgemäßen Schaftprothese 1"' eine Querschnittsgestalt gezeigt, die auf der Grundform eines Rechtecks aufbaut und an allen Kanten abgestufte Anfasungsbereiche 17 aufweist. Die durch die gestrichelte Linie umschriebene Außenkontur stellt dabei skizzenhaft einen herkömmlichen Schaftquerschnitt mit unter 45° zu den Seitenflächen geneigten Anfasungsbereichen für den gleichen Einsatz dar. Es ist zu erkennen, daß die (mit einer durchgezogenen Linie gezeichnete) vorgeschlagene neue Ausführung über den größten Bereich aller Seitenflächen dieser bekannten Ausführung gegenüber übermaßig geschmiedet ist. Alle Anfasungsbereiche haben aber einen mittleren Abschnitt, der mit den Fasen des entsprechenden herkömmlichen Prothesenschaftes deckungsgleich ist. Gegenüber dieser Fase geringfügig nach innen zurückgesetzt und parallel zu ihr verlaufend sind beidseits zu den entsprechenden Seitenflächen hin jeweils Abstufungen vorgesehen.

Eine die in Fig. 13 dargestellte abgestufte Kantengestalt ergebende Nachfräsung hat sich als relativ leicht realisierbar und in vorteilhafter Weise wirkungsvoll erwiesen; grundsätzlich sind aber auch andere Feinstrukturen im Kantenbereich möglich, mit denen die Maßhaltigkeit der Kanten (genauer gesagt: der Fasen) in Einklang gebracht wird mit einer Übermaßigkeit der verbleibenden Seiten- und Stirnflächen - beispielsweise Abrundungen oder zusätzliche, gegenüber der eigentlichen Fase geneigt ausgeführte Anfasungen.

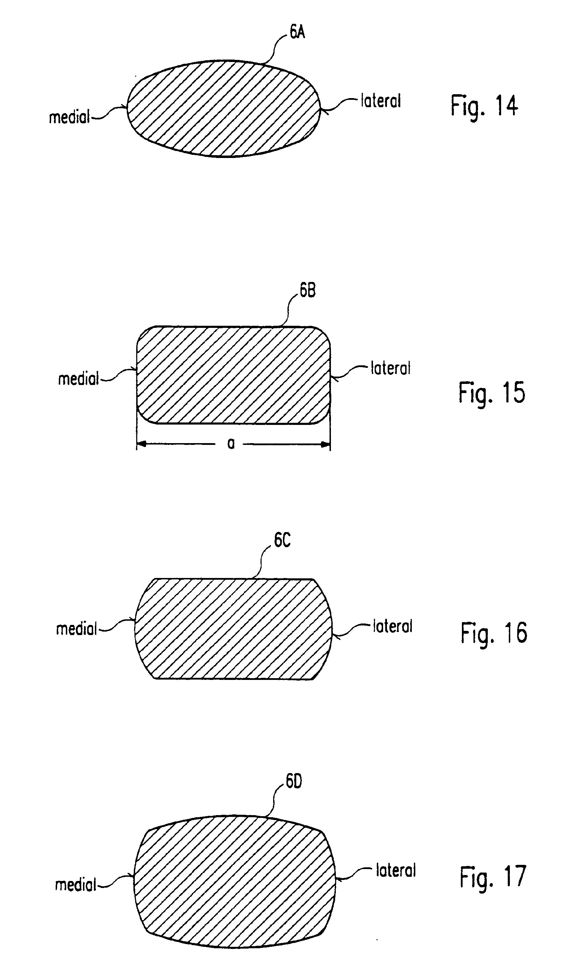

In den Fig. 14 - 17 sind - lediglich als Beispiele für eine Vielzahl möglicher konkreter Querschnittsformen zu verstehen - einige bevorzugte Ausführungen des Prothesenhalses 3 bzw. 3' einer in Fig. 1 und 2 bzw. Fig. 9 dargestellten Hüftgelenkprothese im Querschnitt gezeigt. Zur Unterscheidung voneinander und von dem (im Querschnitt als kreisförmig angenommenen) Prothesenhals 6 bzw. 6' nach Fig. 1 bzw. 9 sind die Prothesenhälse in diesen Figuren mit 6A, 6B, 6C bzw. 6D bezeichnet. Bei der Ausführung nach Fig. 14 ergibt die anterior-posteriore Abflachung einen elliptischen Querschnitt, wobei die große Achse der Ellipse im wesentlichen in medial-lateraler Richtung verläuft. Fig. 15 zeigt eine andere Variante, bei der der Prothesenhals den Querschnitt eines in den Eckbereichen abgerundeteten Rechtecks hat. Die längere Rechtecksseite a erstreckt sich hier - analog zur großen Achse der Ellipse nach Fig. 14 - in medial-lateraler Richtung. Bei der in Fig. 16 gezeigten Ausführung sind die anteriore und posteriore Begrenzungsgrenze der Querschnittsform Geraden, während die mediale und laterale Begrenzungslinie jeweils Kreisbogenabschnitte sind. Der in Fig. 17 gezeigte Prothesenhals 6D hat eine Querschnittsform, bei der alle Begrenzungslinien Kreisbogenabschnitte sind, wobei die die anteriore und posteriore Begrenzung bildenden Kreisbögen einen größeren Radius als die die mediale Begrenzung bildenden Kreisbögen haben.

Es ist leicht einzusehen, daß zwischen den in Fig. 14 - 17 gezeigten und vorstehend kurz beschriebenen Querschnittsformen zahlreiche Mischformen möglich sind, wobei insbesondere gekrümmte Begrenzungslinien auch durch Ellipsen- oder Parabelabschnitte gebildet sein können.

### Bezugszeichenliste

- 1; 1'; 1"; 1"': Schaft
- 2: kragenartiger Ansatz
- 3; 3'; 3": Übergangsbereich
- 4; 4': Zapfen
- 5; 5": distales Schaftende
- 6; 6A; 6B; 6C; 6D: Prothesenhals
- 7a, 7b; 7a", 7b": zusätzliche konische Erweiterung anterior/posterior
- 8; 8'; 8": Übergang von distal zu proximal
- 9; 9': Trochanterflügel
- 10: Bogen
- 11, 12: Bohrung
- 13: mediale Seite distal eben
- 14: mediale Seite proximal spitzdachförmig
- 15: Längsmittellinie des Schaftes
- 15a, 15b, 15c, 15d: Facette
- 16': Erweiterungsbereich
- 17: Anfasungsbereich

- a, b: maximale Erweiterungs-Höhe

- A-A, B-B: Schnittlinie

## Patentansprüche

1. Kombination von blattartigem Schaft einer Hüftgelenkprothese für die Verankerung im Femur und dazugehöriger Raspel, wobei der Schaft (1; 1; 1; 1) sich von einem distalen Ende (5; 5) aus nach proximal allseitig erweitert und medial in einen Bogen übergeht, der sich in einen Prothesenhals (6; 6) fortsetzt,
**dadurch gekennzeichnet,**
**daß** der Schaft in einem proximalen Bereich (8; 8; 8) mindestens in seiner anterior-posterioren Erstreckung ein Übermaß im Bereich zwischen 1 und 3 %, gegenüber dem Maß der größenmäßig dem Schaft entsprechenden Raspel aufweist derart, daß der Schaft beim Eintreiben den Knochen im proximalen Bereich des Femurs flächig verdichtet.

2. Kombination nach Anspruch 1,
**dadurch gekennzeichnet,**
**daß** in dem proximalen Bereich (8; 8; 8) an der anterioren und/oder posterioren Seitenfläche eine zusätzliche Verdickung (7a, 7b; 7a, 7b; 16) vorgesehen ist.

3. Kombination nach Anspruch 2,
**dadurch gekennzeichnet,**
**daß** die zusätzliche Verdickung (7a, 7b; 7a, 7b; 16) in ihrem Längsverlauf an mindestens einer Seite konkav gekrümmt ist.

4. Kombination nach Anspruch 1 oder 2,
**dadurch gekennzeichnet,**
**daß** die zusätzliche Verdickung (7a, 7b; 7a, 7b; 16) in Richtung von distal nach proximal an mindestens einer Seite geradlinig verläuft.

5. Kombination nach einem der Ansprüche 2 bis 4,
**dadurch gekennzeichnet,**
**daß** die zusätzliche Verdickung (7a, 7b; 16) anterior und/oder posterior im Schnitt senkrecht zur Schaftlängsachse (15; 15) spitzdachartig ausgebildet ist.

6. Kombination nach einem der Ansprüche 2 bis 4,
**dadurch gekennzeichnet,**
**daß** die zusätzliche Verdickung anterior und/oder posterior im Schnitt quer zur Schaftlängsachse (15) mindestens abschnittsweise flach konvex oder konkav ausgebildet ist.

7. Kombination nach Anspruch 5 oder 6,
**dadurch gekennzeichnet,**
**daß** die zusätzliche Verdickung (7a, 7b; 7a, 7b; 16) im Schnitt senkrecht zur Schaftlängsachse (15; 15) an einer Seite
- anterior oder posterior - spitzdachförmig oder konvex gewölbt ausgebildet ist, während sie an der anderen Seite
- posterior oder anterior - mindestens abschnittsweise flach konkav oder konkav gewölbt gestaltet ist.

8. Kombination nach einem der vorangehenden Ansprüche,
**dadurch gekennzeichnet,**
**daß** wenigstens eine Seite - medial, lateral, anterior und/oder posterior - im wesentlichen über seine Gesamterstreckung von distal nach proximal im wesentlichen durchgehend spitzdachförmig ausgebildet ist (Fig. 3 bis 8).

9. Kombination nach Anspruch 6,
**dadurch gekennzeichnet,**
**daß** zwei gegenüberliegende Seiten, wahlweise alle Seiten, zumindest bereichsweise, insbesondere im proximalen Bereich, spitzdachförmig ausgebildet sind (Fig. 3 bis 8).

10. Kombination nach einem der Ansprüche 2 bis 9,
**dadurch gekennzeichnet,**
**daß** die zusätzliche Verdickung (7a, 7b) an jeder Seite eine maximale Höhe (a, b) von etwa 2/10 mm bis etwa 10/10 mm, insbesondere 5/10 mm aufweist.

11. Kombination nach einem der vorangehenden Ansprüche,
**dadurch gekennzeichnet,**
**daß** die mediale und/oder laterale Seite im distalen Bereich flach, im proximalen Bereich jedoch spitzdachförmig ausgebildet ist (Fig. 7).

12. Kombination nach einem der vorhergehenden Ansprüche,
**dadurch gekennzeichnet,**
**daß** bei spitzdachförmiger Ausbildung wenigstens einer Seite des Schaftes (1; 1; 1; 1) der Winkel zwischen den beiden Spitzdach-Seiten sich von distal nach proximal ändert, insbesondere stetig zunimmt.

13. Kombination nach einem der vorangehenden Ansprüche,
**dadurch gekennzeichnet,**
**daß** er im distalen und mittleren Bereich und insbesondere auch in den Kantenbereichen des proximalen Bereiches, bezogen auf das Raspelmaß, paßgenau ausgeführt ist.

14. Kombination nach einem der vorhergehenden Ansprüche,
**dadurch gekennzeichnet,**
**daß** die Kanten bzw. Übergangsbereiche zwischen den Seitenflächen des Schaftes (1; 1; 1; 1) mindestens im proximalen Bereich (8; 8; 8) angefast sind unter Ausbildung von Längsfacetten (15a, 15b, 15c, 15d).

15. Kombination nach Anspruch 14,
**gekennzeichnet durch**
abgestufte Anfasungsbereiche (17) an den Kanten des proximalen Bereiches (8; 8; 8), die insbesondere in ihrem mittleren Abschnitt paßgenau in Bezug auf das Maß der größenmäßig zugeordneten Raspel gefräst sind.

16. Kombination nach einem der vorangehenden Ansprüche,
**dadurch gekennzeichnet,**
**daß** der Schaftquerschnitt in einer Ebene senkrecht zur Längsachse (15; 15) eine, insbesondere symmetrische, Trapezform hat, wobei die die mediale Stirnfläche des Schaftes bestimmende Seitenkante des Trapezes kürzer als die die laterale Stirnfläche bestimmende Seitenkante ist.

17. Kombination nach einem der vorangehenden Ansprüche,
**dadurch gekennzeichnet,**
**daß** die zusätzliche Verdickung (7a, 7b; 7a, 7b; 16) durch eine nachträgliche Beschichtung, insbesondere eine Ti-Plasmabeschichtung oder Hydrxylapatitbeschichtung, gebildet ist.

18. Kombination nach Anspruch 17,
**dadurch gekennzeichnet,**
**daß** die zusätzliche Beschichtung einen Mehrschichtaufbau aufweist, der mindestens eine dünnere, dichte erste Schicht und eine auf diese aufgebrachte dickere, poröse zweite Schicht umfaßt.

19. Kombination nach einem der Ansprüche 1 bis 18,
**dadurch gekennzeichnet,**
**daß** der Schaft nahe dem distalen Ende (5; 5; 5) eine polierte Oberfläche und im übrigen Bereich ein rauhe Oberfläche aufweist.

20. Kombination nach Anspruch 19,
**dadurch gekennzeichnet,**
**daß** in einem proximalen Bereich (8; 8; 8) eine stark aufgerauhte, insbesondere offenporige, Oberfläche vorgesehen ist, während in einem mittleren Bereich eine mäßig rauhe, geschlossenporige, insbesondere durch Korundstrahlen erzeugte, Oberfläche vorgesehen ist.

21. Kombination nach einem der Ansprüche 1 bis 20,
**dadurch gekennzeichnet,**
**daß** die maximale Erstreckung des Prothesenhalses (6) quer zu einer Längsachse desselben in anterior-posteriorer Richtung geringer als die maximale Erstreckung in medial-lateraler Richtung ist.

22. Kombination nach Anspruch 21,
**dadurch gekennzeichnet,**
**daß** der Querschnitt des Prothesenhalses (6) im wesentlichen elliptisch oder rechteckig ist oder mindestens eine gerade und eine gekrümmte Begrenzungslinie aufweist.

## Claims

1. Combination of a blade-like stem of a hip joint prosthesis for anchoring in the femur and a rasp associated therewith, wherein the stem (1; 1; 1; 1) extends on all sides from the distal end (5; 5) to the proximal, and merges medially into an arcuate portion, which continues into a prosthesis neck (6; 6),
**characterised in that**
the stem has in a proximal region (8; 8; 8), at least in its anterior-posterior extension, an oversizing in the region of between 1 and 3 % compared with the size of the rasp corresponding in size to the stem so that, when the stem is driven in, it effects compression of the bone over the surface in the proximal region of the femur.

2. Combination according to claim 1,
**characterized in that**
an additional thickened portion (7a; 7b; 7a; 7b; 16) is provided in the proximal region (8; 8; 8) on the anterior and/or posterior lateral face.

3. Combination according to claim 2,
**characterized in that**
the longitudinal course of the additional thickened portion (7a, 7b; 7a, 7b; 16) has a concave curvature on at least one side.

4. Combination according to claim 1 or 2,
**characterized in that**
the additional thickened portion (7a, 7b; 7a, 7b; 16) extends in a straight line in the direction from the distal to the proximal on at least one side.

5. Combination according to any one of claims 2 to 4,
**characterized in that**
in the section perpendicular to the longitudinal axis (15; 15) of the stem, the additional thickened portion (7a, 7b; 16) is anteriorly and/or posteriorly in the shape of a pitched roof.

6. Combination according to any one of claims 2 to 4,
**characterized in that**
in the section transverse to the longitudinal axis (15) of the stem, the additional thickened portion has anteriorly and/or posteriorly, at least in part, a shallow convex or shallow concave shape.

7. Combination according to claim 5 or 6,
**characterized in that**
in the section perpendicular to the longitudinal axis (15; 15) of the stem, the additional thickened portion (7a, 7b; 7a, 7b; 16) is at one side - posteriorly or anteriorly - in the shape of a pitched roof or has convex curvature, whereas at the other side - posteriorly or anteriorly - it has at least in part a shallow convex or shallow concave curvature.

8. Combination according to any one of the preceding claims,
**characterized in that**
at least one side - medially, laterally, anteriorly and/or posteriorly - is essentially over its entire length from the distal to the proximal substantially continuously of pitched-roof shape (Figs. 3 to 8).

9. Combination according to claim 6,
**characterized in that**
two opposite sides, according to choice all sides, are in the shape of a pitched roof at least regionally, especially in the proximal region (Figs. 3 to 8).

10. Combination according to any one of claims 2 to 9,
**characterized in that**
the maximum height (a, b) at each side of the additional thickened portion (7a, 7b) is from approximately 2/10 mm to approximately 10/10 mm, especially 5/10 mm.

11. Combination according to any one of the preceding claims,
**characterized in that**
the medial and/or lateral side is flat in the distal region but of pitched-roof shape in the proximal region (Fig. 7).

12. Combination according to any one of the preceding claims,
**characterized in that**
in the pitched-roof shape arrangement of at least one side of the stem (1; 1; 1; 1), the angle between the two sides of the pitched roof changes, especially steadily increases, from the distal to the proximal.

13. Combination according to any one of the preceding claims,
**characterized in that**
it is arranged to fit exactly in the distal and central region, and especially also in the edge regions of the proximal region, relative to the rasp dimensions.

14. Combination according to any one of the preceding claims,
**characterized in that**
the edges or transition regions between the lateral faces of the stem (1; 1; 1; 1) are bevelled at least in the proximal region (8; 8; 8), forming longitudinal facets (15a, 15b, 15c, 15d).

15. Combination according to claim 14,
**characterized by**
stepped bevelled regions (17) at the edges of the proximal region (8; 8; 8), which, especially in their central portion, are bevelled to be an exact fit relative to the dimensions of the suitably sized rasp.

16. Combination according to any one of the preceding claims,
**characterized in that**
the stem cross-section in a plane perpendicular to the longitudinal axis (15; 15) is in the shape of a trapezium, especially a symmetrical trapezium, the side edge of the trapezium determining the medial face of the stem being shorter than the side edge determining the lateral face.

17. Combination according to any one of the preceding claims,
**characterized in that**
the additional thickened portion (7a, 7b; 7a, 7b; 16) is formed by an after-coating, especially a Ti-plasma coating or hydroxyl apatite coating.

18. Combination according to claim 17,
**characterized in that**
the additional coating has a multi-layer construction, which comprises at least one thinner, dense first layer and, applied to that, a thicker, porous second layer.

19. Combination according to any one of claims 1 to 18,
**characterized in that**
the stem has a polished surface in the vicinity of the distal end (5; 5; 5) and a rough surface in the remaining region.

20. Combination according to claim 19,
**characterized in that**
a markedly roughened, especially open-pore, surface is provided in a proximal region (8; 8; 8), while a moderately roughened, closed-pore surface, produced especially by corundum blasting, is provided in a central region.

21. Combination according to any one of claims 1 to 20,
**characterized in that**
the maximum extent of the prosthesis neck (6) transverse to the longitudinal axis thereof is smaller in the anterior-posterior direction than the maximum extent in the medial-lateral direction.

22. Combination according to claim 21,
**characterized in that**
the cross-section of the prosthesis neck (6) is substantially elliptical or rectangular or has at least one straight and one curved boundary line.

## Revendications

1. Combinaison d'une queue en forme de lame d'une prothèse de la hanche destinée à être ancrée dans le fémur et d'une râpe correspondante, la queue (1 ; 1 ; 1 ; 1) s'élargissant de tous côtés dans la direction proximale à partir d'une extrémité distale (5 ; 5), et aboutissant médialement à un coude prolongé par un col de prothèse (6 ; 6), **caractérisée en ce que**
la queue présente une surépaisseur dans une région proximale (8 ; 8 ; 8) au moins dans son extension antéro-postérieure, comprise entre 1 et 3 % par rapport à l'épaisseur de la râpe de grandeur correspondant à la queue, de telle manière que la queue comprime superficiellement l'os dans la région proximale du fémur lors de l'enfoncement.

2. Combinaison selon la revendication 1, **caractérisée en ce que**
une surépaisseur complémentaire (7a, 7b ; 7a, 7b ; 16) est prévue sur la surface latérale antérieure et/ou postérieure dans la région proximale (8 ; 8 ; 8).

3. Combinaison selon la revendication 2, **caractérisée en ce que**
la surépaisseur complémentaire (7a, 7b ; 7a, 7b ; 16) présente une incurvation concave sur au moins un côté dans le sens de sa longueur.

4. Combinaison selon la revendication 1 ou 2, **caractérisée en ce que**
la surépaisseur complémentaire (7a, 7b ; 7a, 7b ; 16) présente un tracé linéaire sur au moins un côté dans le sens distal-proximal.

5. Combinaison selon l'une des revendications 2 à 4, **caractérisée en ce que**
la surépaisseur complémentaire (7a, 7b ; 16) présente une forme en toit pointu antérieurement et/ou postérieurement en coupe perpendiculaire à l'axe longitudinal de la queue (15 ; 15).

6. Combinaison selon l'une des revendications 2 à 4, **caractérisée en ce que**
la surépaisseur complémentaire présente une forme au moins partiellement faiblement convexe ou concave antérieurement et/ou postérieurement en coupe transversale à l'axe longitudinal de la queue (15).

7. Combinaison selon la revendication 5 ou la revendication 6, **caractérisée en ce que**
la surépaisseur complémentaire (7a, 7b ; 7a, 7b ; 16) présente une forme en toit pointu ou une incurvation convexe sur un côté - antérieur ou postérieur - en coupe perpendiculaire à l'axe longitudinal de la queue (15 ; 15), alors qu'elle présente une incurvation faiblement concave ou concave au moins partiellement sur l'autre côté - postérieur ou antérieur.

8. Combinaison selon l'une des revendications précédentes, **caractérisée en ce que**
au moins un côté - médial, latéral, antérieur et/ou postérieur - présente sensiblement sur toute son extension dans le sens distal-proximal une forme en toit pointu pratiquement continue (fig. 3 à 8).

9. Combinaison selon la revendication 6, **caractérisée en ce que**
deux côtés opposés, optionnellement tous les côtés, présentent une forme en toit pointu au moins partiellement, en particulier dans la région proximale (fig. 3 à 8).

10. Combinaison selon l'une des revendications 2 à 9, **caractérisée en ce que**
la surépaisseur complémentaire (7a, 7b) présente sur chaque côté une hauteur maximale (a, b) comprise entre 2/10^{e} de mm et 10/10^{e} de mm environ, en particulier 5/10^{e} de mm.

11. Combinaison selon l'une des revendications précédentes, **caractérisée en ce que**
le côté médial et/ou latéral est plat dans la région distale, mais présente toutefois une forme en toit pointu dans la région proximale (fig. 7).

12. Combinaison selon l'une des revendications précédentes, **caractérisée en ce que**
en cas de conformation en toit pointu d'au moins un côté de la queue (1 ; 1 ; 1 ; 1), l'angle compris entre les deux pentes du toit aigu varie, en particulier augmente de manière continue dans le sens distal-proximal.

13. Combinaison selon l'une des revendications précédentes, **caractérisée en ce que**
elle est exécutée avec une précision d'ajustement dans la région distale et centrale, et notamment aussi dans les zones de bordure de la région proximale, par rapport à la dimension de la râpe.

14. Combinaison selon l'une des revendications précédentes, **caractérisée en ce que**
les bords ou les zones de transition entre les surfaces latérales de la queue (1 ; 1 ; 1 ; 1) sont chanfreinés au moins dans la région proximale (8 ; 8 ; 8) pour former des facettes longitudinales (15a, 15b, 15c, 15d).

15. Combinaison selon la revendication 14, **caractérisée par**
des zones de chanfreinage étagées (17) sur les bords de la région proximale (8 ; 8 ; 8), lesquelles sont fraisées avec une précision d'ajustement en particulier dans leur partie centrale, par rapport à la dimension de la râpe de grandeur correspondante.

16. Combinaison selon l'une des revendications précédentes, **caractérisée en ce que**
la section transversale de queue présente une forme trapézoïdale, notamment symétrique, sur un plan perpendiculaire à l'axe longitudinal (15 ; 15), le bord latéral du trapèze définissant la surface frontale médiale de la queue étant plus court que le bord latéral définissant la surface frontale latérale.

17. Combinaison selon l'une des revendications précédentes, **caractérisée en ce que**
la surépaisseur complémentaire (7a, 7b ; 7a, 7b ; 16) est formée par un revêtement ultérieur, en particulier un revêtement plasma titane ou un revêtement hydroxylapatite.

18. Combinaison selon la revendication 17, **caractérisée en ce que**
le revêtement complémentaire présente une structure stratifiée comprenant au moins une première couche dense mince et une deuxième couche poreuse épaisse appliquée sur celle-ci.

19. Combinaison selon l'une des revendications 1 à 18, **caractérisée en ce que**
la queue présente une surface polie à proximité de l'extrémité distale (5 ; 5 ; 5), et une surface rugueuse dans les autres parties.

20. Combinaison selon la revendication 19, **caractérisée en ce que**
une surface fortement rugosifiée, en particulier à pores ouverts est prévue dans une région proximale (8 ; 8 ; 8), alors qu'une surface moyennement rugueuse, à pores fermés et produite en particulier par grenaillage au corindon est prévue dans une région centrale.

21. Combinaison selon l'une des revendications 1 à 20, **caractérisée en ce que**
l'extension maximale du col de prothèse (6) transversalement à un axe longitudinal de celui-ci en direction antéro-postérieure est inférieure à l'extension maximale en direction médiale-latérale.

22. Combinaison selon la revendication 21, **caractérisée en ce que**
la section transversale du col de prothèse (6) est sensiblement elliptique ou rectangulaire, ou présente au moins une ligne de délimitation droite et une ligne de délimitation courbe.
